# EUROPEAN PATENT APPLICATION

(11) **EP 4 666 965 A1**
(43) Date of publication of application: **24.12.2025**
(21) Application number: 23946367.2
(22) Date of filing: 20.09.2023
(51) Int. Cl.: A61B 17/16, A61B 17/32

(54) **TAPERED TUBULAR OTOLOGIC BURR**

(30) Priority: 26.07.2023 CN 202321992344 U
(71) Applicant: Bangshi Medical Technology Co., Ltd., Taizhou, Jiangsu 225316 (CN)
(72) Inventor: HE, Chengdong, Taizhou, Jiangsu 225316 (CN); DONG, Qingpeng, Taizhou, Jiangsu 225316 (CN); YUE, Xin, Taizhou, Jiangsu 225316 (CN)
(74) Representative: Bayramoglu et al.
(86) International application number: PCT/CN2023/119908
(87) International publication number: WO 2025/020286

(57) **Abstract**

The present utility model provides a tapered tubular otological drill, and belongs to the technical field of medical appliances. The present utility model aims to solve the technical problem of limited surgical field of view (FOV) in the existing otological drill due to the same diameters of the proximal and distal ends of the drill shank within the same step. The tapered tubular otological drill includes a drill bit, a drill shank assembly and a drill holder, where the drill bit is connected to a connecting rod; the drill shank assembly surrounds the connecting rod; the drill shank assembly includes a connecting rod support, a drill bit support, an inner drill shank, a positioning member and an outer drill shank that are arranged in sequence from inside to outside around the connecting rod; the outer drill shank includes a proximal end and a distal end that are opposite to each other; and the outer drill shank is uniformly tapered from the proximal end to the distal end. The tapered tubular otological drill of the present utility model improves the surgical FOV, reduces the probability of drill leakage, and avoids bouncing of the drill shanks.

## Description

### TECHNICAL FIELD

The present utility model relates to the technical field of medical appliances, and in particular to a tapered tubular otological drill.

### BACKGROUND TECHNOLOGY

It is often necessary to use a medical drill to remove bone tissues from a patient's ear in otologic surgery. For example, during otologic surgery, the drill is driven by a power handle to grind relevant tissues inside the ear of the patient. The typical otological drilling device includes a drill bit, a locking device, and a power handle. Specifically, the locking device is configured to connect and lock the drill bit and the power handle.

At present, the structure of the otological drilling device is basically fixed, and research and development mainly focuses on targeted improvements according to problems encountered by surgeons in actual surgical processes.

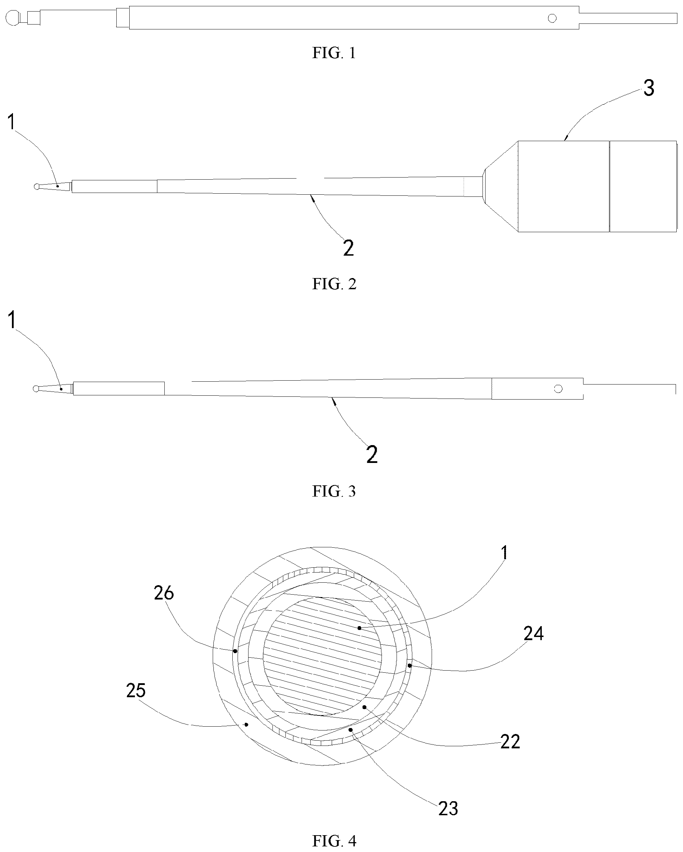

For example, some surgeons reported that the surgical field of view (FOV) of an otological drill was limited after the otological drill is put into actual use. FIG. 1 shows an otological drill in the prior art. As shown in FIG. 1, the entire drill shank assembly is in a stepped cylindrical shape. In practical work, since the proximal and distal ends of the drill shank within the same step have the same diameter, a portion of the surgical FOV is obstructed by the drill shank body. On the other hand, the fabrication of the drill shank assembly of this structure requires multiple process steps and is costly.

### CONTENT OF THE UTILITY MODEL

An objective of the present utility model is to provide a tapered tubular otological drill to solve the technical problem of limited surgical field of view (FOV) in the existing otological drill due to the same diameters of the proximal and distal ends of the drill shank within the same step.

To achieve the above objective, the present utility model provides the following technical solution.

The present utility model provides a tapered tubular otological drill, including a drill bit, a drill shank assembly and a drill holder, where the drill bit is connected to a connecting rod; and the drill shank assembly surrounds the connecting rod; and
the drill shank assembly includes a connecting rod support, a drill bit support, an inner drill shank, a positioning member and an outer drill shank that are arranged in sequence from inside to outside around the connecting rod; the outer drill shank includes a proximal end and a distal end that are opposite to each other; and the outer drill shank is uniformly tapered from the proximal end to the distal end.

The above technical solution has the following technical effects. The outer drill shank is uniformly tapered from the proximal end to the distal end. Compared to the setting where the diameters of the proximal end and the distal end of the drill shank are the same, the present utility model can provide a better surgical FOV to ensure surgical safety. In addition, setting the number of layers of the drill shank to two can reduce the occurrence of water leakage at the weld formed by welding compared to the prior art.

Optionally or preferably, an outer wall of the outer drill shank is provided with a water inlet; a fluid channel is formed between the inner drill shank and the outer drill shank; and the fluid channel allows water from the water inlet to pass through the fluid channel and then flow out from the drill bit.

Optionally or preferably, the positioning member is a semi-tubular elastic member; and the positioning member is provided between the inner drill shank and the outer drill shank and adjacent to the drill bit.

The above technical solution has the following technical effects. The positioning member is provided to limit the inner drill shank and the outer drill shank, avoiding significant vibrations caused by the bouncing of the drill shanks and ensuring surgical safety.

Optionally or preferably, the proximal end of the outer drill shank has a diameter of 3.5 mm, and the distal end has a diameter of 2.2 mm.

According to above technical solutions, embodiments of the present utility model can achieve at least the following technical effects.
(1) In the tapered tubular otological drill provided in the present utility model, the outer drill shank is uniformly tapered from the proximal end to the distal end. Compared to the setting where the diameters of the proximal end and the distal end of the drill shank are the same, the present utility model can provide a better surgical FOV while achieving smooth transition, thereby ensuring surgical safety.
(2) The number of layers of the drill shank changes from four in the prior art to two, reducing water leakage at the weld formed by welding.
(3) The positioning member is added between the inner drill shank and the outer drill shank to limit the inner drill shank and the outer drill shank, avoiding significant vibrations caused by the bouncing of the drill shanks and ensuring surgical safety.

### DESCRIPTION OF THE DRAWINGS

FIG. 1 is a front view of a drill shank assembly of an otological drill in the prior art;
FIG. 2 is a front view of a tapered tubular otological drill according to the present utility model;
FIG. 3 is a front view of a drill shank assembly of the tapered tubular otological drill according to the present utility model;
FIG. 4 is a lateral view of the drill shank assembly of the tapered tubular otological drill according to the present utility model;
FIG. 5 is a front section view of the drill shank assembly of the tapered tubular otological drill according to the present utility model;
FIG. 6 is a partial enlarged view of A shown in FIG. 5; and
FIG. 7 is a partial enlarged view of B shown in FIG. 5.

Reference Numerals: 1. drill bit; 11. connecting rod; 2. drill shank assembly; 21. connecting rod support; 22. drill bit support; 23. inner drill shank; 24. positioning member; 25. outer drill shank; 26. fluid channel; 27. water inlet; and 3. drill holder.

### SPECIFIC IMPLEMENTATIONS

The technical solutions in the embodiments of the present utility model are clearly and completely described below with reference to the drawings in the embodiments of the present utility model. Apparently, the described embodiments are merely a part rather than all of the embodiments of the present utility model. All other embodiments derived from the embodiments in the present utility model by those of ordinary skill in the art without creative efforts should fall within the protection scope of the present utility model.

### Embodiment

As shown in FIGS. 2 to 3, a tapered tubular otological drill includes drill bit 1, drill shank assembly 2, and drill holder 3. The drill bit 1 is connected to connecting rod 11, and the drill shank assembly 2 surrounds the connecting rod 11.

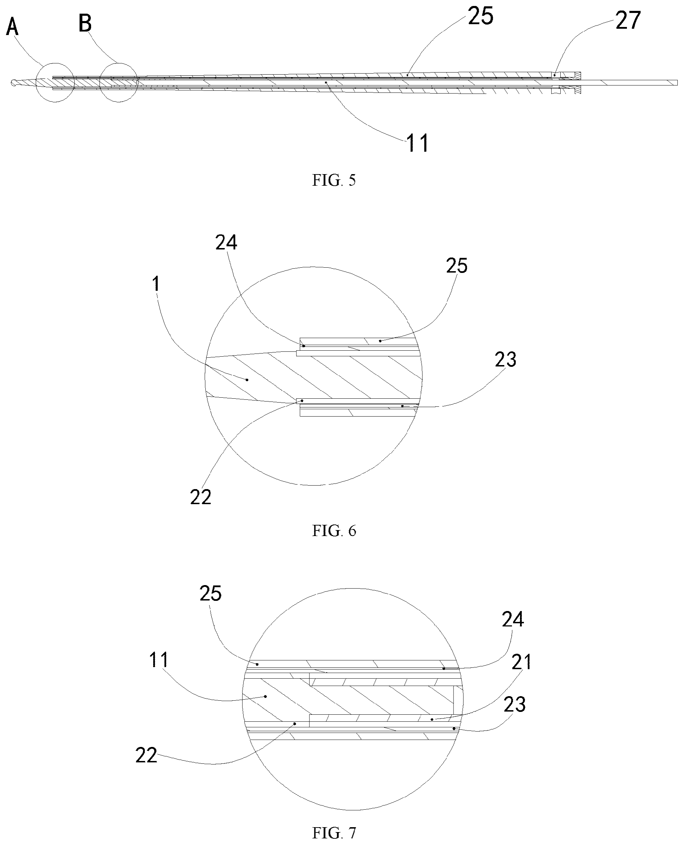

As shown in FIGS. 4 to 7, in this embodiment, the drill shank assembly 2 includes connecting rod support 21, drill bit support 22, inner drill shank 23, positioning member 24, and outer drill shank 25 that are arranged in sequence from inside to outside around the connecting rod 11. The connecting rod support 21 and the drill bit support 22 are respectively configured to limit, fix, and support the connecting rod 11 and the drill bit 1.

In this embodiment, the outer drill shank 25 includes a proximal end and a distal end that are opposite to each other. Regarding the proximal end and the distal end, the proximal end is an end of the otological drill closer to a user, and the distal end is an end of the otological drill farther from the user.

Furthermore, the outer drill shank 25 is uniformly tapered from the proximal end to the distal end. The outer drill shank 25 is integrally formed and has a tapered tube shape. In the tapered tubular design, a diameter of the outer drill shank 25 gradually decreases uniformly from the proximal end to the distal end, achieving a smooth transition and expanding the surgical field of view (FOV) for the user.

In an optional implementation, the proximal end of the outer drill shank 25 has a diameter of 3.5 mm, and the distal end has a diameter of 2.2 mm.

In this embodiment, an outer wall of the outer drill shank 25 is provided with water inlet 27. Fluid channel 26 is formed between the inner drill shank 23 and the outer drill shank 25. In practical work, surgical water enters the fluid channel 26 through the water inlet 27 to flow out from the drill bit 1 to flush the surgical site.

The positioning member 24 is provided between the inner drill shank 23 and the outer drill shank 25 and adjacent to the drill bit 1. The positioning member 24 is configured to limit the inner drill shank 23 and the outer drill shank 25, avoiding significant vibrations caused by the bouncing of the drill shanks.

In this embodiment, the positioning member 24 is a semi-tubular elastic member, with its cross-sectional shape shown in FIG. 4. It is understandable that the water from the water inlet 27 can flow out from an area of the fluid channel 26 not obstructed by the positioning plate 24.

In practical work, the drill bit 1 and the drill shank assembly 3 are fixed inside the drill holder 3. Before the start of a surgery, the drill holder 3 and a power handle are connected and are locked by a locking device. The power handle provides surgical power required by the drill bit 1.

Since this solution does not involve any improvements to the drill holder 3, it does not provide a detailed description of the drill holder 3. Understandably, in this solution, the drill bit 1 and the drill shank assembly 2 can be combined with any drill holder 3 and power handle of known structures to achieve a grinding function.

Compared to the existing otological drill shown in FIG. 1, the present utility model provides a tapered tubular otological drill, in which the outer drill shank 25 is in an integrated tapered tubular shape, with its diameter gradually decreasing uniformly from the proximal end to the distal end. Since the outer drill shank 25 approaches a center, the obstruction of the drill shank on the light at the drill bit 1 is reduced, thereby achieving smooth transition of the drill shank and expanding the surgical FOV.

The number of layers of the drill shank changes from the existing four to two, reducing water leakage at the weld formed by welding.

In addition, the positioning member 24 is added between the inner drill shank 23 and the outer drill shank 25 to limit the inner drill shank and the outer drill shank, avoiding significant vibrations caused by the bouncing of the drill shanks and ensuring surgical safety.

Although the embodiments of the present utility model have been illustrated and described, those of ordinary skill in the art can understand that various changes, modifications, replacements, and alterations may be made to these embodiments without departing from the principle and spirit of the present utility model, and the scope of the present utility model is defined by the claims and equivalents thereof.

## Claims

1. A tapered tubular otological drill, comprising a drill bit (1), a drill shank assembly (2) and a drill holder (3), **characterized in that** the drill bit (1) is connected to a connecting rod (11); and the drill shank assembly (2) surrounds the connecting rod (11); and
the drill shank assembly (2) comprises a connecting rod support (21), a drill bit support (22), an inner drill shank (23), a positioning member (24) and an outer drill shank (25) that are arranged in sequence from inside to outside around the connecting rod (11); the outer drill shank (25) comprises a proximal end and a distal end that are opposite to each other; and the outer drill shank (25) is uniformly tapered from the proximal end to the distal end.

2. The tapered tubular otological drill according to claim 1, **characterized in that** an outer wall of the outer drill shank (25) is provided with a water inlet (27); a fluid channel (26) is formed between the inner drill shank (23) and the outer drill shank (25); and the fluid channel (26) allows water from the water inlet (27) to pass through the fluid channel (26) and then flow out from the drill bit (1).

3. The tapered tubular otological drill according to claim 1, **characterized in that** the positioning member (24) is a semi-tubular elastic member; and the positioning member (24) is provided between the inner drill shank (23) and the outer drill shank (25) and adjacent to the drill bit (1).

4. The tapered tubular otological drill according to claim 1, **characterized in that** the proximal end of the outer drill shank (25) has a diameter of 3.5 mm, and the distal end has a diameter of 2.2 mm.
